(19)

Europäisches
Patentamt
European
Patent Office
Office européen
des brevets

(11) **EP 4 036 864 A1**

(12) **EUROPEAN PATENT APPLICATION**

(43) Date of publication:
**03.08.2022 Bulletin 2022/31**

(21) Application number: **21154231.1**

(22) Date of filing: **29.01.2021**

(51) International Patent Classification (IPC):
**G06T 19/00** $^{(2011.01)}$

(52) Cooperative Patent Classification (CPC):
**G06T 19/00;** G06T 2210/41; G06T 2210/56

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **IBRAGIMOV, Zalman
5656 AE Eindhoven (NL)**
• **SUDARSKY, Oded
5656 AE Eindhoven (NL)**

(74) Representative: **Philips Intellectual Property &
Standards
High Tech Campus 52
5656 AG Eindhoven (NL)**

(54) **GENERATING AN ANATOMICAL MODEL OF AN ANATOMICAL CAVITY**

(57)     A mechanism for synthesizing additional points for generating an anatomical model of an anatomical cavity. The additional points are positioned to lie partway between points directly derived from respective electrical responses of an interventional device positioned within the anatomical cavity.

FIG. 5

EP 4 036 864 A1

**Description**

TECHNICAL FIELD

The present disclosure relates generally to the field of medical imaging, and in particular, to the generation of anatomical models using electrodes mounted on interventional devices.

BACKGROUND

Medical imaging is an important aspect in diagnosing and/or treating a subject. There is an increasing interest in the use of dielectric imaging, sometimes referred to as electroanatomical mapping, processes for imaging or modelling of an anatomical cavity and/or structure. Dielectric imaging processes facilitate high quality imaging or modelling of a subject's anatomy without the need for contrast agents and/or X-ray.

In a dielectric imaging process, two or more crossing electrical fields are induced by an array of electrodes positioned on the outside of the subject ("external electrodes"), such as on the surface of the subject. These electric fields induce position dependent electromagnetic responses, such as a voltage response, in electrodes placed within the body (an "internal electrode"). A mapping function is used to predict or "map" the position dependent electromagnetic response to a position in space. The position of internal electrodes can thereby be tracked by monitoring the response of the electrodes to these electric fields.

By tracking and iteratively recording the position of the internal electrodes, a model of the internal anatomy of the subject can be (re)constructed, e.g. by constructing a mesh around the recorded positions of the internal electrodes. This is because it can be generally assumed that the internal electrodes will only be located within cavities of the subject, thereby allowing the bounds of the cavity to be constructed.

One example of a suitable mechanism for constructing a model of the internal anatomy of the subject based on the response of internal electrodes to crossing electric fields induced by external electrodes is disclosed by the European Patent Application having the publication number EP 3568068 A1.

There is an ongoing desire to improve the accuracy of models constructed using the predicted positions of the internal electrodes.

SUMMARY OF THE INVENTION

The invention is defined by the claims.

There is proposed a computer-implemented method of generating an anatomical model of an anatomical cavity into which an interventional device, comprising one or more electrodes, is positioned.

The computer-implemented method comprises: obtaining, for each of a plurality of positions of the interventional device within the anatomical cavity and from each electrode of the interventional device, an electrical response of the respective electrode to electric fields induced within the anatomical cavity; and defining, for each obtained electrical response, a point in a Euclidian space system based on the electrical response; and for each of one or more sets of the defined points, synthesizing one or more additional points in the Euclidian space system based on the set of defined points, each additional point lying partway between two or more of the defined points; and generating an anatomical model, of the anatomical cavity, by processing the defined points and the one or more synthesized additional points, wherein each set of the defined points comprises either: points defined using electrical responses obtained at a same position of the interventional device; or points defined using temporally adjacent electrical responses of a same electrode,

The present disclosure proposes an approach for increasing or densifying the number of points from which an anatomical model is generated. In particular, additional points are synthesized that lie between existing, "true" points directly derived from respective electrical responses of the interventional device.

The disclosure relies upon a recognition that such intermediate or interpolated points will have a high likelihood of lying within the anatomical cavity (due to the limitation of the interventional device), and can therefore be used to generate the anatomical model without significantly affecting its accuracy. Indeed, the more points that are available for generating the anatomical model, the more accurately the anatomical model represents the anatomical cavity. The advantage gained in providing additional points outweighs any disadvantage in using simulated or synthetic points.

There is therefore proposed an approach for increasing an accuracy of the anatomical model. Approaches also facilitate generation of an anatomical model at an earlier point in time than previously available (as, typically, a minimum number of points are required before an anatomical model can be constructed).

In some examples, the computer-implemented method further comprises a step of outputting a representation of the anatomical model to a user interface. This approach provides a clinician with useful clinical information (about the anatomical cavity) to aid them in performing a medical procedure, such as a diagnosis or assessment.

In some examples, an electrical response comprises two or more values, each value representing a measurement of an electrical parameter responsive to changes in a different electric field induced within the anatomical cavity. Thus, an electrical response may effectively represent a point within a response space. Examples of suitable electrical parameters will be readily apparent to the skilled person - e.g. a voltage measurement (e.g. voltage measurement being specific to a particular electric field, e.g. by way of frequency).

In some examples, the step of defining, for each electrical response, a point in the Euclidian space system comprises defining the point as having co-ordinates equal to the two or more values of the electrical response.

In other examples, the step of defining, for each electrical response, a point in the Euclidian space system comprises using a mapping function to map the obtained electrical response to a point in the Euclidian space system, wherein the mapping function is configured such that that predetermined properties and/or spatial relationships of the electrodes are maintained. Thus, each point may represent a point in physical or "real" space.

Optionally, each axis of the Euclidian space system represents a predicted position within a physical space or a position space

In some examples, the step of generating an anatomical model comprises processing the defined points and the one or more synthesized additional points to define the position of the bounds of the anatomical cavity within a 3D volume of discrete voxels representing the Euclidian space system. Thus, the anatomical model may be represented using a 3D matrix of values (defining the bounds of the anatomical cavity within a particular position/physical space), each value representing a particular voxel of the 3D volume.

In some examples, the length of each side of each voxel is the same and equal to a voxel size, wherein: the step of synthesizing one or more additional points comprises synthesizing one or more additional points such that each additional point meets first predetermined constraints.

The first predetermined constraints may comprise constraints that: the distance between the additional point and any of the set of the defined points is no less than the voxel size; and the distance between the additional point and any other additional point synthesized from the same set of defined points is no less than the voxel size.

The voxel size defines the precision of the anatomical model. It is here recognized that there is little purpose in generating additional points that are spaced more closely than the voxel size, as these will increase the complexity of generating/constructing the anatomical model without improving the precision or accuracy of the anatomical model.

Preferably, step of synthesizing the one or more additional points comprises synthesizing the maximum number of additional points that adhere to the first predetermined constraints. This approach maximizes the potential accuracy of the anatomical model by providing the most efficient number of additional points for densifying the points used to generate the anatomical model.

The step of synthesizing one or more additional points may comprise synthesizing one or more additional points such that each additional point, synthesized from the same set of defined points, is evenly spaced between the said set of defined points. This provides an easy to implement option that provides an even distribution of additional points. A distribution evenness of points facilitates a higher quality anatomical model.

The interventional device may be an elongate device comprising two or more electrodes at different positions along the elongate device. In one example, each set of the defined points consists of defined points representing the electrical responses of two adjacent electrodes of the interventional device. The additional point(s) generated from the set of defined points may be placed upon a hypothetical line spanning between the points representing the two adjacent electrodes of the interventional device. This provides a low-complexity approach to calculating the appropriate position(s) for the additional points.

The interventional device may comprise a hoop-shaped portion upon which two or more electrodes are mounted. In such examples, each set of the defined points comprises points lying in a same plane and/or lying substantially in a circle. Thus, sets of defined points may only be established if they are sufficiently circularly arranged and/or arranged in a same plane. This avoids the generation of inaccurate additional points (e.g. those that would lie outside the bounds of the anatomical cavity).

There is also proposed a computer program product comprising computer program code means which, when executed on a computing device having a processing system, cause the processing system to perform all of the steps of any herein claimed method.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

There is also proposed an anatomical model generator configured to generate an anatomical model of an anatomical cavity into which an interventional device, comprising one or more electrodes, is positioned.

The anatomical model generator comprises: an input interface configured to obtain, for each of a plurality of positions of the interventional device within the anatomical cavity and from each electrode of the interventional device, an electrical response of the respective electrode to electric fields induced within the anatomical cavity.

The anatomical model generator also comprises a data processor configured to: define, for each obtained electrical response, a point in a Euclidian space system based on the electrical response; and for each of one or more sets of the defined points, synthesize one or more additional points in the Euclidian space system based on the set of defined points, each additional point lying partway between two or more of the defined points; and generate an anatomical model, of the anatomical cavity, by processing the defined points and the one or more synthesized additional points, wherein each set of the defined points comprises either: points defined using electrical responses obtained at a same position of the interventional device; or points defined using temporally adjacent electrical responses of a same electrode.

The anatomical model generator may comprise an output interface, configured to output the anatomical model, or a representation thereof, to a user interface, e.g. for display.

There is also proposed an anatomical model system comprising an anatomical model generator as previously described and a user interface configured to display a visual representation of the anatomical model generated by the anatomical model generator. The user interface may receive the anatomical model or a representation thereof from the output interface of the anatomical model generator.

There is also proposed a processing system comprising the anatomical model generator or the anatomical model system previously described.

The processing system further may further comprise an electric field generator, configured to control the electric fields induced within the anatomical cavity. The electric field generator may communicate with one or more electrodes (positioned on a surface of the subject) in order to generate the electric fields. The processing may itself comprise the electrodes that generate the electric fields.

In some examples, the processing system comprises an electrode response sensing device, configured to sense or sample one or more electrical responses of the electrodes of the interventional device to the electric fields. The electrode response sensing device may be communicatively coupled to the electrodes of the interventional device (e.g. by one or more wires) and configured to sample the one or more electrical responses. In particular, the electrode response sensing device may be configured to sense or sample, at each of a plurality of time points, a set of measurements from each electrode of the interventional device (the set of measurements forming the electrical response of each electrode of the interventional device).

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates positions for a set of external electrodes positioned on a subj ect;
Figure 2 illustrates a co-ordinate system for defining a direction of electric fields generated by the external electrodes;
Figure 3 illustrates a dielectric imaging system;
Figure 4 illustrates an approach for reconstructing an anatomical model from a point cloud;
Figure 5 illustrates a method according to an embodiment;
Figure 6 illustrates an interventional device;
Figure 7 illustrates another interventional device; and
Figure 8 illustrates an anatomical model generator.

DETAILED DESCRIPTION OF THE EMBODIMENTS

The invention will be described with reference to the figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

The invention provides a mechanism for synthesizing additional points for generating an anatomical model of an anatomical cavity. The additional points are positioned to lie partway between points directly derived from respective electrical responses of an interventional device positioned within the anatomical cavity.

The present disclosure is based on a realization that hypothetical points that lie between true or existing points (e.g. in a response space or position/physical space) will have a high likelihood of also lying within the anatomical cavity, i.e. representing possible points that could plausibly be derived from the interventional device. Thus, these additional points could be used to generate the anatomical model without significantly affecting the accuracy of the anatomical model and/or increase an accuracy of the anatomical model (especially during early instances of generating the anatomical model, when fewer "true" measured points are available).

Embodiments of the invention may be employed in any suitable scenario in which an anatomical model of an anatomical cavity is generated based on the response(s) of one or more internal electrodes (mounted on an interventional device) to electric fields induced within the anatomical cavity. One example scenario could be the mapping of a heart or chambers thereof and surrounding blood vessels using an interventional device such as a catheter inserted into the cardiovascular system.

For the purposes of contextual understanding, the principle and purpose of generating an anatomical model of an anatomical cavity, involving the use of electric fields within a subject, will be hereafter described.

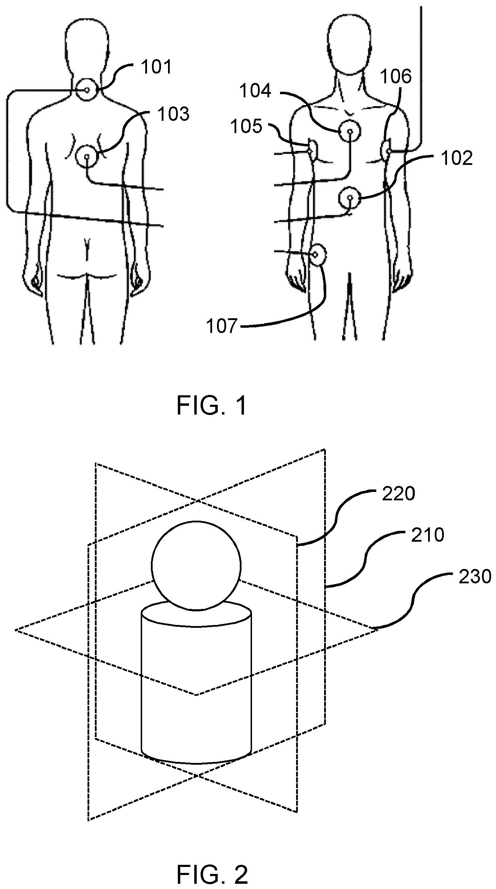

Figure 1 illustrates positions for a set of external electrodes positioned on a subject 190. External electrodes are electrodes that are positioned externally to a subject, and are contrasted with internal electrodes that are positionable within the subject, for example as part of an interventional device such as a catheter.

The illustrated set of external electrodes comprises a first external electrode 101, a second external electrode 102, a third external electrode 103, a fourth external electrode 104, a fifth external electrode 105 and a sixth external electrode 106. These electrodes are preferably located on the subject such that they can provide crossing fields within the subject as described hereinafter. The illustrated set further comprises a reference electrode 107, which can act as a "ground" for defining a base/background level of electric field activity in the subject.

An electric signal provided to each external electrode is controlled to thereby define crossing electric fields between the electrodes. In particular, the first 101 and second 102 external electrodes form a first pair of external electrodes ("external electrode pair"), and signals provided to the first external electrode pair can be controlled to induce a first electric field therebetween. The third 103 and fourth 104 external electrodes form a second pair of external electrodes, and signals provided to the second external electrode pair can be controlled to induce a second electric field therebetween. The fifth 105 and sixth 106 external electrodes form a third pair of external electrodes, and signals provided to the third external electrode pair can be controlled to induce a third electric field therebetween. The pairs of electrodes are preferably arranged such that they provide substantially mutually orthogonal crossing fields.

Electric signals provided to each external electrode, and in particular to each pair of electrodes, can control the frequency and/or magnitude/strength of the crossing electric fields. The electric signals supplied to the electrodes may be controlled by an electric field generator (not illustrated in Figure 1).

The crossing electric fields are usable to track or identify a location of electrodes positioned within the crossing electric fields. In particular, an electrical response of an electrode to each electric field will change as a position within the electric field changes (e.g. a distance from a source/sink of the electric field changes). The electrical response of an electrode to the crossing electric fields can be mapped or associated with a particular position within the electric fields.

In other words, an electrical response (which can be alternatively labelled a "measurement") of the internal electrode to the crossing electric fields can be processed to determine a predicted position of the internal electrode (e.g. and therefore of any interventional device comprising the internal electrode) within an anatomical cavity of the subject.

In particular, each electric field may be controlled to have a particular/different frequency. This facilitates determination of the predicted position of an electrode with respect to each electric field, by assessing the electrical response of the electrode to the particular frequency of the electric field. This information can be used to effectively identify or predict the position of the electrode with respect to a co-ordinate system defined by the electric fields.

One of the external electrode pairs may be omitted, e.g. if only two crossing electric fields are desired (e.g. for performing a 2-dimensional tracking process).

Figure 2 schematically illustrates a co-ordinate system for defining a direction of electric fields generated by the external electrodes. The co-ordinate system defines three cardinal planes 210, 220, 230. The crossing electric fields may, for instance, be optimally positioned when the direction of each electric field is parallel to a respective cardinal place 210, 220, 230.

The intent of the crossing electric fields, e.g. such as those generated using the set illustrated by Figure 1, is to facilitate identification of the position of an electrode (or group of electrodes) with respect to a co-ordinate system, such as that illustrated by Figure 2.

A more complete example of how to track the position of an internal electrode with respect to crossing electric fields how to further exploit this information for the construction of an anatomical model of an anatomical cavity of the subject, is hereafter described for the purposes of improved contextual understanding.

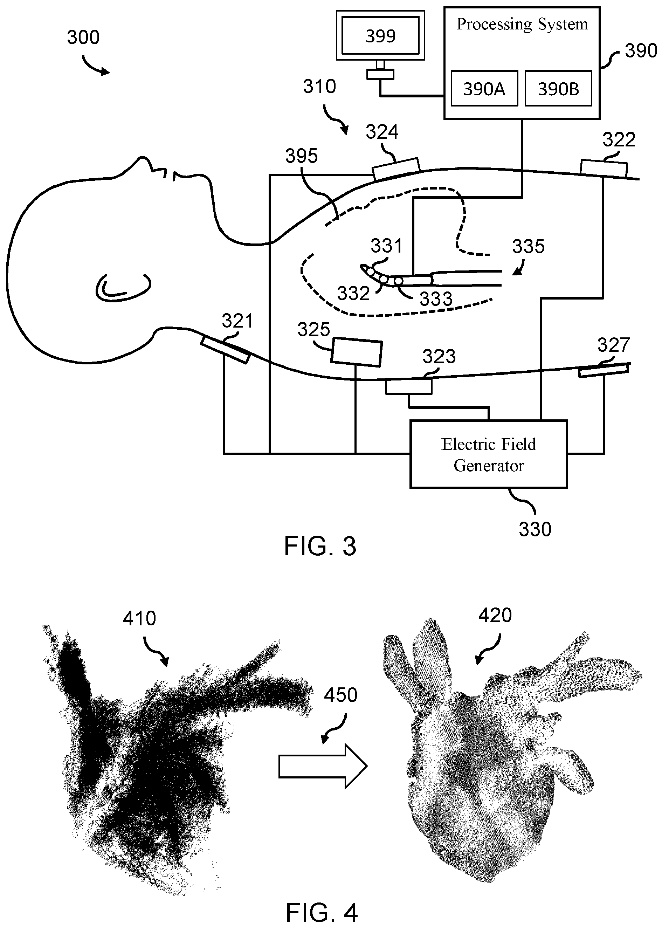

Figure 3 conceptually illustrates a processing arrangement 300 for generating a mapping function for predicting the position of an electrode within an anatomical cavity and for generating an anatomical model of an anatomical cavity within a subject. The concept of the present disclosure is primarily directed towards the process of generating the anatomical model.

In particular, the processing arrangement 300 may be configured to generate an anatomical model (of an anatomical cavity, such as a blood vessel and/or chamber) using a dielectric imaging process sometimes also referred to as an anatomical mapping process and the arrangement may be called a dielectric imaging system or an anatomical mapping system.

The dielectric imaging system 300 comprises an exemplary electric field generating arrangement 310 and a processing system 390. The processing system 390 may contain a mapping function generator 390A (i.e. a "processor") and/or an anatomical model generator 390B according to an embodiment, although each of these elements may be alternatively positioned externally to the processing system 390 (e.g. part of the electric field generator). Although not separately shown, the processing system 390 may also comprise a electric field measurer (i.e. an electrode response sampling device) that is controlled by the processing system to detect/measure electrical signals of the electrodes of the interventional device.

The processing system may therefore also comprise an electric signal measurer (not shown) configured to measure and/or sample the electrical response(s) of the electrode(s) of the interventional device to crossing electric fields, as explained in more detail below.

In some examples, the processing system 390 may perform at least some of the functions of an electric field generator 330, described in more detail below.

The electric field generating arrangement 310 comprises a set of external electrodes 321, 322, 323, 324, 325, 327 for being positioned externally with respect to the subject 390 (e.g. as electrode patches provided on a skin of the subject). The set 310 of external electrodes may comprise a plurality of electrode pairs angled with respect to one another (e.g. positioned orthogonally to one another), so that any electric fields generated by the electrode pairs are angled with respect to one another. These electrode pairs may comprise a first electrode pair (formed of a first 321 and second 322 external electrode), a second electrode pair (formed of a third 323 and fourth 324 external electrode) and a third electrode pair (formed of a fifth 325 and sixth (not visible) external electrode). One of more of these electrode pairs may be omitted. The set of external electrodes may also comprise a reference electrode 327. Thus, the external electrodes are placable in a similar manner to the set of external electrodes illustrated in Figure 1.

The electric field generating arrangement 310 also comprises an electric field generator 330, which is adapted to generate and/or control (characteristics of) an electric signal (e.g. voltage and/or current) supplied to each external electrode. The electric field generator may, in some example, form part of the processing system 390.

The electric field generating arrangement 310 is configured to generate a plurality of (here: three) crossing (intra-body) electrical fields using the external electrodes. This is performed using appropriate control of the electric signals provided to each external electrode for example alternating constant currents such as constant amplitude currents are provided to each of the electrode pairs.

In particular, each electrode pair may be appropriately controlled to induce an electric field between each electric pair. Thus, where there are three electrode pairs, three electric fields may be generated. Preferably, the frequency of each generated electric field is controlled to be different, to facilitate increased ease in identifying a relative location of an electrode positioning within the crossing electric fields.

The generated electric fields can be used to define or establish a relative location of an (internal) electrode positioned within the crossing electric fields. In particular, as previously explained, the (electrical) response of an internal electrode to the electric fields changes as the predicted position of the internal electrode moves about the subject. This is at least partly because the induced electrical fields' distribution is inherently inhomogeneous due to the different dielectric properties and absorption rates (related to conductivity) of the interrogated tissues of the subject under investigation. The principle of crossing electric fields thereby facilitates tracking of the predicted position of the internal electrode using an anatomical model generator 390 that monitors an electrical response of any internal electrodes to the crossing electric fields, e.g. by mapping the (electrical) response of an electrode to a predicted position within the subject or using a suitable mapping/transfer function, e.g. a "V2R function" as for example described below to determine the predicted position(s) of the electrodes.

By way of further explanation, for the purposes of improved conceptual understanding, if the crossing electric fields are controlled to have a different frequency with respect to one another, then the electrical response of the internal electrode to each frequency could be used to determine a relative distance between each source/sink of the corresponding electric field. This principle can be used to effectively triangulate the predicted position of the internal electrode within the crossing electric fields.

For instance, if there are three external electrode pairs, positioned to emit electric fields ($E_1$, $E_2$, $E_3$) of different frequencies that are angled (e.g. near-orthogonal) with respect to one another, a voltage response ($V_1$, $V_2$, $V_3$) of an internal electrode (identifying a voltage (e.g. between the electrode and the reference electrode or between the electrode and the electrode generating the electric field) at each of these three frequencies) will differ depending upon position within the anatomical cavity.

Thus, an electrical response of an electrode may comprise two or more values (e.g. voltage measurements), each value representing a measurement of an electrical parameter responsive to changes of a different electric field induced within the anatomical cavity. Here, each value represents a voltage measurement at a particular frequency (being the frequency of the corresponding electric field). Other suitable electrical measurements could be used instead.

The skilled person will appreciate that determining the position of internal electrodes 331, 332, 333 also facilitates determining the position, orientation and/or angle of an interventional device 325 that mounts the electrodes. In particular, a positional relationship of the electrodes on the interventional device may be known/predetermined, and used to derive an orientation of the interventional device (e.g. define an axis along which the interventional device lies).

Other forms of electrical response for an internal electrode (e.g. an impedance response or a capacitive response, e.g. indicating change in impedance/capacitance between the internal electrode and an external electrode) will be apparent to the skilled person. In such examples, each value of the response may represent an impedance/capacitance measurement at a particular frequency or with respect to a particular electrode emitting a particular electric field.

The electric field generator 330 may be configured to control the electric fields, generated using the set of external electrodes, to operate in the frequency range of 10-100 kHz, e.g. between 10kHz and 25kHz. These frequency ranges is particularly useful for ensuring penetration of a subject, whilst providing a frequency range that attenuates in human tissue without significant damage/injury to the tissue. A lower frequency range (e.g. between 10kHz and 25kHz, e.g. between 10kHz and 20kHz) also benefits from a reduced impact of noise. This facilitates the measurement of more localized and precise electrical responses, with reduced interruption from external sources. The electrical response of two or more internal electrodes mounted upon a single interventional device can be used to construct (and update) the mapping function used to map an electrical response of the internal electrode to a predicted position within the anatomical cavity (e.g. within some Euclidian/Cartesian space).

An exemplary process for generating or constructing a mapping function using a mapping function generator 390A is hereafter described.

As previously mentioned, the mapping function generator makes use of a plurality of internal electrodes 331, 332, 333 to be positioned within the anatomical cavity (e.g. electrodes positioned on an interventional device 335 to be inserted into the anatomical cavity). A spatial relationship (e.g. a distance) between each of the internal electrodes may be predetermined and/or known.

The mapping function generator 390A is configured to receive (and optionally provide) signals (e.g. at an input interface) from/to the internal electrodes 331, 332, 333 to determine an electrical response of the internal electrodes to the crossing electric fields.

The electrical response of the internal electrode(s) (e.g. to externally applied electric fields by the external electrodes) is/are then iteratively recorded or sampled for different positions and/or orientations of the interventional device within the body cavity explored, i.e. to obtain or sample one or more "measurements" from each internal electrode. For instance, each sampled response may contain three measurements each corresponding to a respective electric field. This sampling may be performed by an electrode response sampling device (not shown), which may form part of the processing system 390.

It will be understood that this generates a temporal sequence of sets of one or more electrical responses, each set of one or more electrical responses being obtained at a same point in time (i.e. when the interventional device is at a same position).

Conceptually, each electrical response may itself represent a "point" (e.g. a location defined by a single set of co-ordinates) within a particular Euclidian space system, i.e. a "response space". Where the electrical response is a voltage response, this may be known as the "V-space". The electrical response may, for instance, comprise two or more values/measurements each responsive to a respective (e.g. and single one of the) electric fields (e.g. filtered by frequency of the electric field). Each value may represent a co-ordinate value in the response space. For instance, if each value represents a voltage measurement (at a respective frequency of a specific electric field), then each electrical response can be represented by a point in a voltage space.

The mapping function generator can then repeatedly define/update and apply a mapping function or transfer function (e.g. a "V2R function") that transforms each recorded electrical response to Euclidian/Cartesian coordinates in "real", position/physical space (R-space), whilst ensuring known properties and/or spatial relationships of the internal electrodes and/or interventional device 335 (e.g. electrode spacing and electrical weight length) and, optionally, a set of other constraints are maintained.

A position space, physical space or R-space represents a "true" position or physical space, i.e. where a distance between two different points of an axis of the R-space directly represents a physical distance. This differs from the response space, in which a distance between two different points of an axis does not directly represent a physical distance (but rather, represents a change in an electric parameter).

In some examples, the mapping/transfer function can effectively learn and linearize the distorted relation between the measured responses and the actual position in three dimensions by taking the known inter-electrode distances as a reference. By way of example only, if an internal electrode has an electrical response matching a previously measured electrical response then the relative distance to the electrical responses measured by the other internal electrodes can be determined and used to improve the mapping/transfer function.

In other words, the mapping/transfer function effectively determines or predicts a relative/predicted location of each electrode in a Euclidian/Cartesian and/or multidimensional space or co-ordinate system ("R-space") representing a position/physical space. Thus, the mapping function transfers from a response space to a position/physical space. In this way, an R-space cloud of points (known Euclidian/Cartesian co-ordinates) can be built up and updated as the internal electrodes are moved within the anatomical cavity. This R-space cloud of points is then iteratively analyzed in order to iteratively update the mapping function to adhere to the known properties of the internal electrodes and/or interventional device, such as a spatial relationship of the internal electrodes.

A more detailed description of examples of the aforementioned process may be found in WO2018130974. Other and/or more complete descriptions and/or embodiments of the process for generating the mapping function are disclosed by the European Patent Applications EP 0775466 A2, EP 3568068 A1 and EP 3607879 A1. Other example mapping function may, for instance, make use of machine-learning.

The predicted positions of an internal electrode or internal electrodes (within a position space or R space) may also be used to construct an anatomical model of an anatomical cavity (i.e. a cavity in which the interventional device is able to move). This process is called a reconstruction process (or a dielectric imaging process), and may be performed by the anatomical model generator 390B.

Broadly, the anatomical model generator 390B may build an anatomical model of an anatomical cavity 395 (e.g. a chamber, vessel or void) within a subject by processing an R-space cloud of points. An anatomical model may, for instance, be in the form of a mesh that defines an estimated surface of the anatomical cavity. Other forms of anatomical models would be apparent to the skilled person, e.g. points defining a shape of the anatomical model.

In particular, using an updated R-space cloud of points, such as the R-space cloud of points produced during the construction of the mapping function, a reconstruction algorithm generates an anatomical model of the (investigated part of the) anatomical cavity. The anatomical model may, for example, be a 3D surface that depicts or models the (known bounds of) the anatomical cavity. This may be in the form of a mesh.

In particular, generating an anatomical model may comprise processing the R-cloud of points to define the position of the bounds of the anatomical cavity within a 3D volume of discrete voxels representing the Euclidian space system. The length of each side of each voxel is the same and equal to a voxel size.

In particular, the determined points in the physical space may be used to define the bounds of an anatomical cavity within a 3D physical space, thereby defining an anatomical model. Put another way, the anatomical model may be represented by a 3D volume of discrete voxels, e.g. in the form of a 3D matrix, which define the bounds of the anatomical cavity.

Other examples may (further) use an R-space cloud of points obtained using another interventional device (i.e. and not necessary the same device used to generate the mapping function).

The process of reconstructing an anatomical model from a point cloud is conceptually illustrated in Figure 4, which demonstrates a process 450 in which a cloud of R-space points 410 ("point cloud") is transformed into an anatomical model 420. In the illustrated example, this is performed by creating a (3D) surface from the point cloud data, e.g. a mesh, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

The above-provided description of a dielectric imaging process is only an example, and the skilled person would be readily capable of modifying the described process appropriately.

The skilled person will appreciate that the derived mapping/transfer function ("V2R function") can also be used to track a location of the internal electrodes with respect to a constructed anatomical model. This facilitates the generation and display of an anatomical model and an indicator of a current location/position of an interventional device 335 mounting internal electrodes 331, 332, 333 with respect to the anatomical model.

Of course, a visual representation of any generated anatomical model and/or determined position may be generated and provided at a user interface 399.

The present disclosure proposes a new approach for generating the anatomical model based on the electrical responses of electrodes positioned within the anatomical cavity. In particular, the present disclosure proposes the synthetization of new points (in the response space and/or the "real" space, i.e. the R-space), which are used to generate the anatomical model.

The synthetization of new points effectively densifies the point cloud used to generate the anatomical model. This is through direct densification of the R-space points cloud, or the response space point cloud (which new synthesized points are mapped into R-space before generation of the anatomical model).

The additional points generated are positioned to lie partway between two or more defined points, being points that are directly associated with a real measurement obtained from the electrode(s) of the interventional device.

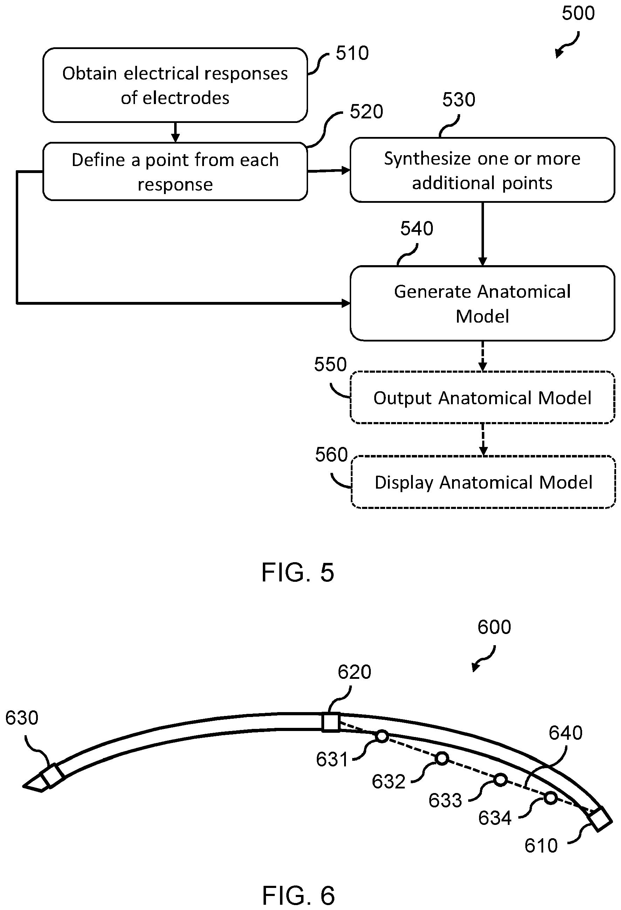

Figure 5 illustrates a (computer-implemented) method 500 according to an embodiment. The method 500 may be performed by the anatomical model generator 390B of Figure 3.

The method 500 comprises a step 510 of obtaining, for each of a plurality of positions of the interventional device within the anatomical cavity and from each electrode of the interventional device, an electrical response of the respective electrode to electric fields induced within the anatomical cavity.

The method 500 comprises a step 520 of defining, for each obtained electrical response, a point in a Euclidian space system based on the electrical response.

Step 520 may comprise, for instance, directly defining a point in the response space (previously described). For instance, if an electrical response comprises two or more values (e.g. each representing an electrical measurement for a respective electric field), then the values may form the co-ordinates for the corresponding point in a response space (an example of a Euclidian space system).

In another example, step 520 comprises applying a mapping function to each obtained electrical response to define a point in the R-space (i.e. a physical space). Embodiments for the mapping function have been previously described.

The method then moves to a process 530 of for each of one or more sets of the defined points, synthesizing one or more additional points in the Euclidian space system based on the set of defined points, each additional point lying partway between two or more of the defined points.

Thus, new points are synthesized (i.e. generated) that are disposed between pre-existing points (i.e. points that directly represent an obtained response of an electrode). These pre-existing points may be labelled "electrode-based points", as they are directly derived by sensing measurements at an electrode.

The synthetic points are effectively interpolated between the electrode-based points, i.e. to be positioned between the electrode-based points. The synthesized additional points, generated from a given set of defined points, are preferably evenly spaced between the points of the given set, i.e. at equal distances in the Euclidian space system.

Each set of the defined points comprises either: points defined using electrical responses obtained at a same position of the interventional device; or points defined using temporally adjacent electrical responses of a same electrode.

In other words, each set of the defined points may comprise points associated with electrical responses of different electrodes that are sampled at a same time (i.e. when the interventional device is at a same position) or points associated with electrical responses of a same electrode sampled at adjacent points in time.

The method 500 may then perform a step 540 of generating an anatomical model, of the anatomical cavity, by processing the defined points and the one or more synthesized additional points. Processes for generating an anatomical model using points are well known and established, such as those previously described with reference to Figure 3. Thus, a conventional approach for constructing an anatomical model can be used that treats the synthesized additional points as points for construction of the anatomical model.

Where the synthesized additional points are generated in the response space, step 540 may comprise using a mapping function to map all defined (electrode-based) and synthesized points to the R-space (i.e. a physical space) before generating the anatomical model. Of course, this process is not necessary if the synthesized additional points are directly generate in the R-space, i.e. in the physical space.

The present invention operates on an underlying assumption that underlying assumption is that, since the electrodes are guaranteed to be inside the anatomical cavity, then positions between the electrode-based points are also likely to be in the anatomical cavity. Thus, additional points can be synthesized. The more points that are available for constructing the anatomical model, the greater the accuracy and/or precision of the anatomical model.

It has previously been explained how points can be generated in the response space or the physical space (i.e. the R-space). However, it is possible that additional points can be synthesized in both the response and physical space, e.g. by performing step 530 for the points in the response space and repeating step 530 for points in the physical space.

Earlier in this disclosure, it was explained how the anatomical model may define the position of the bounds of the anatomical cavity within a 3D volume of discrete voxels representing the Euclidian space system. In other words, the anatomical model may be represented by a 3D volume of discrete voxels (e.g. as a 3D matrix).

The voxel may be in the form of a cube, having equal length sides that are equal to a voxel size. A voxel size thereby defines the (maximum) resolution of the anatomical model. One working example of a voxel size is 1mm, although other examples are contemplated (e.g. 0.5mm, 2mm, 0.25mm and so on). Preferably, the voxel size is no less than 2mm (for providing a sufficiently precise representation of the anatomical structure for the purpose of medical interpretation).

In some preferred examples, when step 530 comprises synthesizing additional points in the physical space, the additional points may be synthesized such that each additional point meets certain (first) predetermined constraints. These predetermined constraints may include constraints that the distance between the additional point and any of the set of the defined points is no less than the voxel size; and the distance between the additional point and any other additional point synthesized from the same set of defined points is no less than the voxel size.

Thus, the distance between any of the defined points (i.e. the electrode-based points) and the synthesized points may be no less than the voxel size of the anatomical model (to be generated from the points). This embodiment recognizes that smaller distances between the synthesized points would add complexity to the reconstruction algorithm without increasing an accuracy or precision of the anatomical model (due to the discretization to the voxel size).

Preferably, the maximum number of additional points that meet the first predetermined constraints are generated. Even more preferably, all additional points are evenly spaced between the set of points from which they are synthesized.

As one working example, a set of defined points may comprise only two points.

In one scenario, a first point (of the set) may be a point defined from an electrical response obtained from a first electrode of the interventional device and a second point (of the set) may be a point defined from an electrical response obtained from a second electrode of the interventional device, where the electrical responses are obtained at a same position of the interventional device (e.g. sampled at a same time).

In another example, a first point (of the set) may be a point defined from an electrical response obtained from a first electrode of the interventional device at a first point in time, and a second point may be a point defined from an electrical response obtained from the same first electrode at a second point in time, where the second point in time is immediately after the first point in time (e.g. in a temporal sequence of points in time at which responses of electrodes are sampled).

Continuing the working example, the additional points may then be synthesized by spacing additional points evenly and linearly between the two points of the set - i.e. interpolating additional points. The distance between the additional points may, for instance, be limited to no greater than a predetermined value (e.g. a voxel size of an anatomical model to be constructed therefrom).

The value(s) of the additional points can be directly determined from the value(s) of the set of defined points. For instance, if each point is defined as having a number of values (representing a position in a particular space), then a first point (of the set) may have values $V_1$ (e.g. $V_{1x}$, $V_{1y}$, $V_{1z}$) and a second point (of the set) may have values $V_2$ (e.g. $V_{2x}$, $V_{2y}$, $V_{2z}$). An additional point may be calculated as having values:

$$V_a = V_x + k\left(\frac{V_y - V_x}{M}\right) \tag{1}$$

The value for M is selected to define a distance between two each additional point, where k ranges from 1 to M-1 and changes for each additional point derived from the same set of defined points.

In scenarios in which the anatomical model is modelled as a 3D volume of discrete voxels, the value for M may be calculated by dividing the distance between the two points of the set by the voxel size, and rounding (e.g. down) the resultant number to the nearest integer.

As a working example, consider a scenario in which the Euclidian co-ordinate system is a physical space, the voxel size of the anatomical model is 1 mm and the (Euclidean) distance between the two defined points (of the set) is 5.1 mm. In this scenario, the additional points are positioned at 1.02, 2.04, 3.06 and 4.08 mm along a hypothetical line spanning between the two defined points.

Although the above approach makes use of linearly positioning additional points between (two) defined points, some embodiments may use non-linear (e.g. curved or making use of higher order polynomials) to define the positions of the additional points.

This approach is only a working example, and other approaches for synthesizing additional points based on a set of known (electrode-based or "real" points) will be apparent to the skilled person.

For instance, a set of defined points may define a plane or volume (e.g. the points bound a particular volume). Additional points may be placed within this plane or volume.

Some further examples will be described below.

In some examples, method 500 may further comprise a step 550 of outputting the generated anatomical model or a representation of the generated anatomical model (e.g. for display by a user interface). In some further examples, the method 500 also comprises a step 560 of displaying the output anatomical model, or a representation of the same, at a user interface.

Figure 6 illustrates an interventional device 600 for use with an embodiment, and is used to conceptually illustrate an example approach proposed by the present disclosure.

The interventional device 600 comprises an elongate portion having two or more electrodes 610, 620, 630 at different positions along the elongate portion. For a given position of the interventional device 600 within an anatomical cavity, an electrical response of each electrode can be sampled, and used to define respective points within a Euclidian co-ordinate system (e.g. a response space or a physical space). Where the Euclidian co-ordinate system represents a physical space (e.g. an R-space), then the position of the electrodes is spatially accurate. Thus, each electrode can be accurately represented (i.e. in data form) by a point in space.

The method 500 is then performed. Here, the obtained electrical responses for the purposes of method 500 are the electrical responses from the electrodes of the interventional device.

For this embodiment, the method 500 is configured such that each set of the defined points consists of defined points representing the electrical responses of two adjacent electrodes of the interventional device (at a particular point in time, e.g. for a particular position of the interventional device). In particular, the synthetization of additional points can be performed between every adjacent pair of electrodes on the interventional device.

Applying method 500 generates a plurality of additional points between each defined point. Figure 6 illustrates the synthesis of four additional points 631, 632, 633, 634 between a set of points derived from two adjacent electrodes 610, 620 of the interventional device 600. The method 500 is configured to linearly position the additional points between the adjacent electrodes, e.g. space the additional points along a hypothetical line 640 spanning between the two points in the set. This approach may make use of equation (1) and the process describing said equation.

The synthesis of the additional points may be linear even if (e.g. as illustrated) the interventional device may not be linear between the electrodes mounted thereon. This disconnect between non-linearity (of the interventional device) and linearity (of the additional points) is not significant, as the distance between linear and higher-order additional points is negligible relative to the precision of the anatomical model (e.g. the voxel size) and because the inter-electrode spacing is significantly smaller than the catheter's minimum radius of curvature.

Of course, high order interpolation and arc length distances can be used instead of linear interpolation and Euclidean distances, respectively.

In scenarios in which the anatomical model is modelled as a 3D volume of discrete pixels, the distance between each pair of points may be no less than the voxel size.

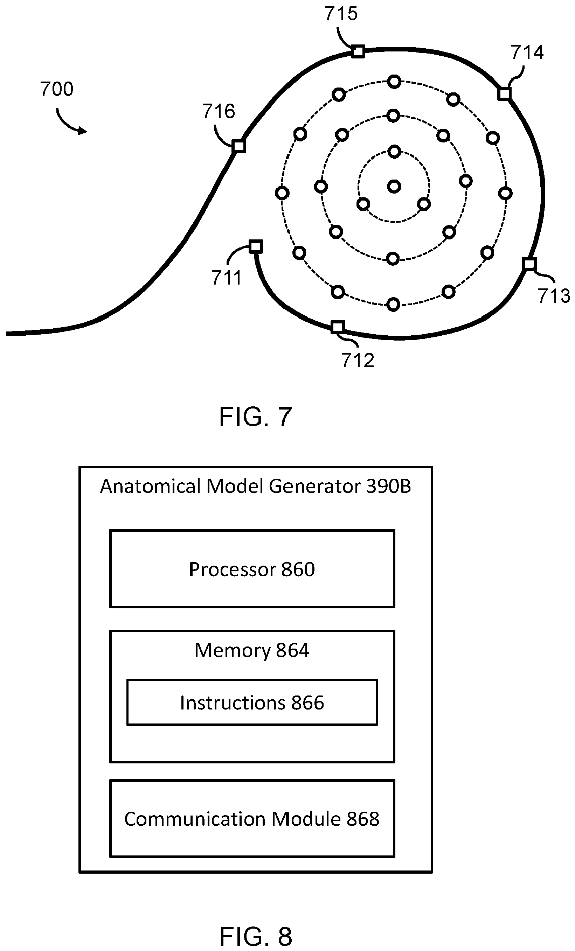

Figure 7 illustrates an interventional device 700 for use with another embodiment, and is used to conceptually illustrate an example approach proposed by the present disclosure.

The interventional device 700 comprises a hoop-shaped portion upon which two or more electrodes 711, 712, 713, 714, 715, 716 are mounted. Such interventional devices are sometimes labelled "radial-type" interventional devices.

As with the interventional device 600 of Figure 6, for a given position of the interventional device 700 within an anatomical cavity, an electrical response of each electrode can be sampled, and used to define respective points within a Euclidian co-ordinate system (e.g. a response space or a physical space). Where the Euclidian co-ordinate system represents a physical space (e.g. an R-space), then the position of the electrodes is spatially accurate.

The method 500 is then performed to generate additional points. For the sake of clarity, the additional points are not individually labelled, but are instead represented by circles. This compares to the electrodes, which are represented by squares. Here, the obtained electrical responses for the purposes of method 500 are the electrical responses from the electrodes of the interventional device 700.

For this embodiment, the method 500 is configured such that each set of the defined points consists of defined points representing the electrical responses of the electrodes of the interventional device 700 for a single position of the interventional device.

In this example, each set of defined points may comprise points lying in a same plane and arranged in a circle (i.e. so that a hypothetical curve connected the points of the set is substantially circular). In other words, each set of defined points.

In other words, each set of defined points may be a set of points that meets the following conditions: a) the points are derived from electrical responses samples for a single, same position of the interventional device; b) the points are arranged in substantially the same plane; and c) the points are arranged substantially circularly.

One approach to determining conditions b) and c) is hereafter described. Other approaches would be apparent to the skilled person (e.g. processing the points using some machine-learning method or other predetermined algorithm).

Firstly, estimate the normal to an average plane of the set of points (e.g. a best fit plane) as the average of the cross products of pairs of consecutive vectors between each point and the center of the points in the set (the "point-center vectors"). The center may be defined as the average of all points in the set. For example, where a point represents coordinates in space, the average co-ordinates. Then, for each point in the set, calculate the distance from the center as the length of the point-center vector (thereby determining a "point-center distance" for each point in the set). The approach also calculates, for each point in the set, a "point-plane distance" (i.e. a distance between the point and the best fit plane) by calculating the dot product between the point-center vector and the normal to the plane. The points in the set may be determined to meet conditions b) and c) if the sum of the ranges of point-center distances and point-plane distances falls below some predetermined threshold. As one example, where the points are points in a physical space, this predetermined threshold may be less than 5mm, e.g. 3mm.

One approach to inserting additional points within the circle defined by the set of points meeting conditions a), b) and c) is hereafter described. In this scenario, it is again assumed that the anatomical model is modelled as a 3D volume of discrete voxels having sides equal to a voxel size, and that the points are located in a physical space.

Round (e.g. down) the average radius to the nearest multiple of the voxel size, to produce a first number. Define concentric, equidistant (hypothetical) circles around the center in the average plane, the number of circles being equal to the first number minus one. In Figure 7, the hypothetical circles are represented in dashed form. Round (e.g. down) every circle's circumference to the nearest multiple of the voxel size, to produce (for each circle) a second number. Position an additional point at equal angular distances on each circle separately, the number of additional points on each circle being equal to the second number for that circle. Finally, position an additional point at the center of the circle, i.e. a 0-radius circle.

This approach inserts additional points that meet the first predetermined constraints, previously described, such that the distance between the additional point and any of the set of the defined points is no less than the voxel size; and the distance between the additional point and any other additional point synthesized from the same set of defined points is no less than the voxel size. This approach also maximizes the number of additional points that are provided and that meet the first predetermined criteria.

Other approaches for positioning additional points based on a set of points will be apparent to the skilled person.

For instance, a set of points may comprise points defined using electrical responses obtained at a same position of the interventional device. These points may define a volume (e.g. a volume having vertices at the defined points). Additional points may be added inside this volume and/or on the surface of this volume. The additional points may meet the first predetermined constraints previously described.

Previous embodiments have focused upon the generation of additional points based on sets of points derived from electrical responses of electrodes sampled at a same time (i.e. for a same position of the interventional device). This approach may be labelled "spatial addition". This approach is acceptable if the length of time between successive samples (i.e. successive acquisitions of the electric responses) is less than some predetermined number (e.g. occurs more frequency than a rate of 100Hz). That is because the distance that an electrode travels (e.g. during 10 milliseconds) at typical sampling speeds is expected to be significantly smaller than the precision of the anatomical model (e.g. where appropriate the voxel size).

However, in some examples, it may be desired to generate additional points based on sets of points derived from electric responses of a same electrode at different (but sequentially adjacent) points in time. In other words, additional points may be generated using a set of points defined using temporally adjacent electrical responses of a same electrode. This approach may be labelled "temporal addition".

In the context of this application "temporally adjacent" means two adjacent samples of a same electrode.

In this way, a set of points can be defined using a sequence of at least two points, the sequence being derived from the electrical response of a same electrical at consecutive/adjacent samples.

This approach adopted for defining the additional points can be much the same as described with reference to Figure 6, e.g. by linearly spacing additional points along a hypothetical line connecting the two points.

In another approach, the set of points may define a plane or volume with a Euclidian co-ordinate space, and the additional points can be positioned to lie within (or on) the defined plane or volume.

It is possible to also generate further additional points using this temporal-based approach based on any additional points generated from a set of points derived from electrical responses obtained at same time (e.g. for a same position of the interventional device).

In particular, each additional point may correspond to another additional point generated from a set of points derived from electrical responses obtained at a second same time (temporally sequential to the time at which the set of points for deriving the said each additional point

Put another, consider a scenario in which a first set of points (derived from the electrical responses of electrodes for a first position of the interventional device) is used to generate a first group of additional points and a second set of points (derived from the electrical responses of electrodes for a second position of the interventional device, the first and second positions being temporally adjacent to one another - such that the first and second set of points are temporally sequential).

An additional point generated from the first set of points will correspond to an additional point generated from the second set of points. These two points could form a further set of points from which further additional points could be generated, e.g. using any previously described approach.

For improved processing efficiency, it may be preferable to not perform specific calculations for setting the distance between additional points for every set of points. Rather, these calculations could be performed in advance - e.g. based on information contained in the technical specification for the interventional device, and/or only once (e.g. upon obtaining a predetermined number of electrical responses and/or points derived therefrom).

In this scenario, for interventional devices having a hoop portion (and which follow the process described with reference to Figure 7), the position of the additional points may be scaled by the ratio between the current radius of the hoop portion (i.e. the radius of the hoop portion when the responses, from which the set of points are derived, were originally sampled) and the radius used to calculate the position of the additional points (i.e. the radius resulting from the calculations performed in advance).

This approach takes account of the recognition that the radius of the hoop will change over time, and that the position of the additional points would (if using a predetermined radius only) therefore may not accurately be positioned between the set of points used to generate the additional points.

Figure 8 is a schematic diagram of an anatomical model generator 390B, according to embodiments of the present disclosure. The anatomical model generator 390B may be adapted to carry out any method herein described.

As shown, the anatomical model generator 390B may include a (data) processor 860, a memory 864, and a communication module 868. These elements may be in direct or indirect communication with each other, for example via one or more buses.

The processor 860 may include a central processing unit (CPU), a digital signal processor (DSP), an ASIC, a controller, an FPGA, another hardware device, a firmware device, or any combination thereof configured to perform the operations described herein. The processor 860 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 864 may include a cache memory (e.g., a cache memory of the processor 860), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 864 includes a non-transitory computer-readable medium. The non-transitory computer-readable medium may store instructions. For example, the memory 864, or non-transitory computer-readable medium may have program code recorded thereon, the program code including instructions for causing the anatomical model generator 390B, or one or more components of the anatomical model generator 390B, particularly the processor 860, to perform the operations described herein. For example, the anatomical model generator 390B can execute operations of the method 700. Instructions 866 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 864, with the code recorded thereon, may be referred to as a computer program product.

The communication module 868 can include any electronic circuitry and/or logic circuitry to facilitate direct or indirect communication of data between the processing system 390, the penetration device and/or the user interface (or other further device). In that regard, the communication module 868 can be an input/output (I/O) device. In some instances, the communication module 868 facilitates direct or indirect communication between various elements of the anatomical model generator and/or the system (Figure 3). Thus, the communication module 868 comprises at least an input interface for receiving electrical responses (e.g. directly from the electrodes or from a memory).

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1.  A computer-implemented method (500) of generating an anatomical model (420) of an anatomical cavity into which an interventional device (335, 600, 700), comprising one or more electrodes (331, 332, 333, 610, 620, 630, 711, 712, 713, 714, 715, 716), is positioned, the computer-implemented method comprising:

    obtaining (510), for each of a plurality of positions of the interventional device within the anatomical cavity and from each electrode of the interventional device, an electrical response of the respective electrode to electric fields induced within the anatomical cavity; and

    defining (520), for each obtained electrical response, a point in a Euclidian space system based on the electrical response; and

    for each of one or more sets of the defined points, synthesizing (530) one or more additional points (631, 632, 633, 634) in the Euclidian space system based on the set of defined points, each additional point lying partway between two or more of the defined points; and

    generating (540) an anatomical model, of the anatomical cavity, by processing the defined points and the one or more synthesized additional points,

    wherein each set of the defined points comprises either: points defined using electrical responses obtained at a same position of the interventional device; or points defined using temporally adjacent electrical responses of a same electrode,

    optionally wherein the computer-implemented method further comprises outputting (550) a representation of the anatomical model to a user interface.

2.  The computer-implemented method of claim 1, wherein an electrical response comprises two or more values, each value representing a measurement of an electrical parameter responsive to changes in a different electric field induced within the anatomical cavity.

3.  The computer-implemented method of claim 2, wherein the step (520) of defining, for each electrical response, a point in the Euclidian space system comprises defining the point as having co-ordinates equal to the two or more values of the electrical response.

4.  The computer-implemented method of any of claims 1 or 2, wherein the step (520) of defining, for each electrical response, a point in the Euclidian space system comprises using a mapping function to map the obtained electrical response to a point in the Euclidian space system, wherein the mapping function is configured such that that predetermined properties and/or spatial relationships of the electrodes are maintained.

5.  The computer-implemented method of claim 4, wherein each axis of the Euclidian space system represents a predicted position within a position space.

6.  The computer-implemented method of any of claims 1 to 5, wherein the step (540) of generating an anatomical model comprises processing the defined points and the one or more synthesized additional points to define the position of the bounds of the anatomical cavity within a 3D volume of discrete voxels representing the Euclidian space system.

7.  The computer-implemented method of claim 6, when dependent upon any of claims 4 or 5, wherein the length of each side of each voxel is the same and equal to a voxel size, and wherein:
    the step (530) of synthesizing one or more additional points comprises synthesizing one or more additional points such that each additional point meets first predetermined constraints, the first predetermined constraints comprising constraints that:

    the distance between the additional point and any of the set of the defined points is no less than the voxel size; and
    the distance between the additional point and any other additional point synthesized from the same set of defined points is no less than the voxel size.

8.  The computer-implemented method of claim 7, wherein the step of synthesizing (530) the one or more additional points comprises synthesizing the maximum number of additional points that adhere to the first predetermined constraints.

9.  The computer-implemented method of any of claims 1 to 8, wherein the step (530) of synthesizing one or more additional points comprises synthesizing one or more additional points such that each additional point, synthesized from the same set of defined points, is evenly spaced between the said set of defined points.

10. The computer-implemented method of any of claims 1 to 9, wherein the interventional device (335, 600) is an elongate device comprising two or more electrodes (331, 332, 333, 610, 620, 630) at different positions along the elongate device, and each set of the defined points consists of defined points representing the electrical responses of two adjacent electrodes (610, 620) of the interventional device.

11. The computer-implemented method of any of claims 1 to 9, wherein the interventional device (700) comprises a hoop-shaped portion upon which two or more electrodes (711, 712, 713, 714, 715, 716) are mounted, and each set of the defined points comprises points lying in a same plane.

12. The computer-implemented method of claim 11, wherein each set of the defined points comprises points lying substantially in a circle.

13. A computer program product comprising code which, when executed by a processor circuit, causes the processor circuit to perform the steps of the method according to any of claims 1 to 12.

14. A non-transitory computer-readable medium or data carrier comprising or carrying the computer program product of claim 13.

15. An anatomical model generator (390B) configured to generate an anatomical model (420) of an anatomical cavity into which an interventional device (335, 600, 700), comprising one or more electrodes (331, 332, 333, 610, 620, 630, 711, 712, 713, 714, 715, 716), is positioned, the processing circuit comprising:

   an input interface (868) configured to obtain (510), for each of a plurality of positions of the interventional device within the anatomical cavity and from each electrode of the interventional device, an electrical response of the respective electrode to electric fields induced within the anatomical cavity; and
   a data processor (860) configured to:

      define (520), for each obtained electrical response, a point in a Euclidian space system based on the electrical response; and
      for each of one or more sets of the defined points, synthesize (530) one or more additional points (631, 632, 633, 634) in the Euclidian space system based on the set of defined points, each additional point lying partway between two or more of the defined points; and
      generate (540) an anatomical model, of the anatomical cavity, by processing the defined points and the one or more synthesized additional points,

   wherein each set of the defined points comprises either: points defined using electrical responses obtained at a same position of the interventional device; or points defined using temporally adjacent electrical responses of a same electrode.

FIG. 1

FIG. 2

FIG. 3

FIG. 4

FIG. 5

FIG. 6

700

715

714

716

711

712

713

FIG. 7

Anatomical Model Generator 390B

Processor 860

Memory 864

Instructions 866

Communication Module 868

FIG. 8

Europäisches
Patentamt

European
Patent Office

Office européen
des brevets

# EUROPEAN SEARCH REPORT

Application Number

EP 21 15 4231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/173697 A1 (ANGIOMETRIX CORP [US]; PATIL NITIN [US] ET AL.) 20 December 2012 (2012-12-20) * paragraphs [0033] - [0035], [0051], [0068], [0084], [0229], [0235], [0236], [0279], [0282], [0292], [0410] * ----- | 1-15 | INV. G06T19/00 |
| X | EP 3 200 159 A1 (BIOSENSE WEBSTER (ISRAEL) LTD [IL]) 2 August 2017 (2017-08-02) * abstract * * paragraphs [0004] - [0006], [0010], [0011] * ----- | 1-10, 13-15 | |

TECHNICAL FIELDS
SEARCHED    (IPC)

G06T

The present search report has been drawn up for all claims

| Place of search | Date of completion of the search | Examiner |
|---|---|---|
| Munich | 15 June 2021 | Eckert, Lars |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 21 15 4231

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2021

| Patent document cited in search report | | Publication date | Patent family member(s) | | | Publication date |
|---|---|---|---|---|---|---|
| WO 2012173697 | A1 | 20-12-2012 | AU | 2012271236 | A1 | 16-01-2014 |
| | | | BR | 112013031673 | A2 | 06-12-2016 |
| | | | CA | 2839158 | A1 | 20-12-2012 |
| | | | CN | 103732132 | A | 16-04-2014 |
| | | | EP | 2717759 | A1 | 16-04-2014 |
| | | | JP | 5911570 | B2 | 27-04-2016 |
| | | | JP | 6114853 | B2 | 12-04-2017 |
| | | | JP | 2014530639 | A | 20-11-2014 |
| | | | JP | 2016165471 | A | 15-09-2016 |
| | | | WO | 2012173697 | A1 | 20-12-2012 |
| EP 3200159 | A1 | 02-08-2017 | AU | 2016277628 | A1 | 17-08-2017 |
| | | | CA | 2955962 | A1 | 28-07-2017 |
| | | | CN | 107085861 | A | 22-08-2017 |
| | | | EP | 3200159 | A1 | 02-08-2017 |
| | | | IL | 250011 | A | 30-06-2020 |
| | | | JP | 6837847 | B2 | 03-03-2021 |
| | | | JP | 2017142788 | A | 17-08-2017 |
| | | | US | 2017221254 | A1 | 03-08-2017 |
| | | | US | 2019213777 | A1 | 11-07-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3568068 A1
- WO 2018130974 A
- EP 0775466 A2
- EP 3607879 A1

**Non-patent literature cited in the description**

- **BERGER, MATTHEW et al.** A survey of surface reconstruction from point clouds. *Computer Graphics Forum,* 2017, vol. 26 (1